# EUROPEAN PATENT SPECIFICATION

(11) **EP 1 550 474 B1**
(45) Date of publication and mention of the grant of the patent: **11.03.2009**
(21) Application number: 03764160.2
(22) Date of filing: 10.07.2003
(51) Int. Cl.: A61M 5/158, A61M 5/32

(54) **INJECTION NEEDLE AND LIQUID INTRODUCING INSTRUMENT**
INJEKTIONSNADEL UND FLÜSSIGKEITSEINFÜHRENDES INSTRUMENT
AIGUILLE D'INJECTION ET INSTRUMENT D'INTRODUCTION DE LIQUIDE

(30) Priority: 10.07.2002 JP 2002201899
(43) Date of publication of application: 06.07.2005
(73) Proprietor: TERUMO KABUSHIKI KAISHA, Tokyo 151-0072 (JP)
(72) Inventor: NISHIKAWA, Hisao, Terumo kabushiki kaisha, Ashigarakami-gun, Kanagawa 259-0151 (JP); OOYAUCHI, Tetsuya, Terumo kabushiki kaisha, Ashigarakami-gun, Kanagawa 259-0151 (JP); YATABE, Teruyuki, Terumo kabushiki kaisha, Ashigarakami-gun, Kanagawa 259-0151 (JP)
(74) Representative: TBK-Patent
(86) International application number: PCT/JP2003/008781
(87) International publication number: WO 2004/006996

(56) References cited:
- EP-A- 1 188 456
- WO-A-02/058769
- WO-A1-92/20389
- DE-A1- 2 408 852
- DE-A1- 4 109 442
- JP-A- 7 313 597
- JP-A- 9 276 403
- JP-U- 52 112 192
- US-A- 4 781 691

## Description

### Technical Field

The present invention relates to an injection needle and a liquid introducing instrument for use in injecting a liquid such as liquid medication into a living body.

### Background Art

Conventional injection needles that are generally used for the self-injection of insulin are relatively thick hollow needles having an outside diameter of 0.25 mm or greater. Injection needles which have outside diameters ranging from 0.3 mm to 1.2 mm and an outside diameter of about 2 mm in some cases are used for normal injection. Such a thick injection needle makes the patient feel pain and hurt when it pierces the patient. Using a thick injection needle for self-administering medication also gives the patient fear and anxiety.

Injection needles which are thinner than the above injection needles for making the patient feel less pain are of lower mechanical strength. Since such thin injection needles necessarily have a reduced inside diameter and a relatively large length, they pose a very large flow passage resistance when liquid medication is injected into the living body. Therefore, a considerable force needs to be applied to push out the liquid medication when it is injected.

Attempts have been made to study an injection needle having a thinner distal end section for piercing the patient, a thicker proximal end section for introducing liquid medication, and a tapered section interconnecting the thinner distal end section and the thicker proximal end section. However, the outer profile of the tapered section is liable to give a burden to the patient when the injection needles is pierced, and the inner profile of the tapered section tends to increase the flow passage resistance.

Post published document WO 02/058769 A discloses a liquid injection needle which includes a hollow needle part for injecting a liquid and a supporting part, to which the needle part is fixed. The needle part includes an anchoring part that extends through the inside of the supporting part and a puncturing part that extends from the supporting part for making a puncture in a living body. The puncturing part has a distal side outer diameter equal to or greater than 0.1 mm and equal to or less than 0.25 mm, and a proximal side outer diameter greater than the distal side outer diameter.

DE 41 09 442 A1 discloses a steel syringe for spinal anaesthesia comprising a straight cylindrical tube into which a removable stylet is inserted, an insertion point, and a front aperture for the tube passage. The external tube diameter and the diameter of the passage taper steadily at the forward end on which the point is situated, the latter and the aperture being at the apex of the cone formed. DE 41 05 442 A1 discloses the combination of features of the preamble of claim 1.

It is the object of the invention to provide an injection needle which is capable to reduce a pain exerted to a patient, which has an improved mechanical strength and poses a very small flow passage resistance.

The object is achieved by an injection needle according to claim 1. Advantageous embodiments are carried out according to the dependent claims.

According to the present invention, there is provided an injection needle comprising a puncture section having a needle point capable of piercing a living body, a proximal end section having outside and inside diameters greater than the puncture section, and a tapered section interconnecting the puncture section and the proximal end section, wherein
the tapered section provides puncture resistance smaller than the puncture section.

According to the present invention, the tapered section has an outer profile having an angle ranging from 0.5° to 1°50' with respect to a line parallel to a central axis of the injection needle.

According to the present invention, the puncture section has an outside diameter ranging from 0.1 mm to 0.5 mm, and the length from the puncture section to the tapered section ranges from 0.2 mm to 15 mm.

According to preferred embodiments of the present invention, there is also provided a liquid introducing instrument for being mounted on a liquid inlet port formed on a distal end of a liquid container that is capable of holding a liquid therein, as defined in claims 3 and 4.

### Brief Description of Drawings

FIG. 1 is a cross-sectional view of an injection needle according to the present invention;
FIG. 2 is a side elevational view of a liquid introducing instrument which is constructed of the injection needle according to the present invention and a base body supporting the injection needle;
FIG. 3 is a cross-sectional view of the liquid introducing instrument according to the present invention shown in FIG. 2;
FIG. 4 is a cross-sectional view of a liquid medication injecting device on which the liquid introducing instrument according to the present invention is mounted;
FIG. 5 is a cross-sectional view of a liquid medication container shown in FIG. 4;
FIG. 6 is a cross-sectional view of a liquid medication injecting device according to another embodiment on which a liquid introducing instrument according to the present invention is mounted;
FIG. 7A is a view illustrative of a first ground angle α of a facet formed on the needle point of the injection needle according to the present invention, FIG. 7B is a view illustrative of a second ground angle Φ of a facet formed on the needle point of the injection needle according to the present invention, FIG. 7C is a cross-sectional view taken along line a - a of FIG. 7A, illustrating a cross-sectional angle γ formed between both ridges of a cross section of the needle point;
FIG. 8 is a line graph showing the results of measured puncture resistance of injection needles; and
FIG. 9 is a diagram of a system for measuring the flow passage resistance of an injection needle.

### Best Mode for Carrying out the Invention

Embodiments of the present invention will be described below with reference to the drawings.

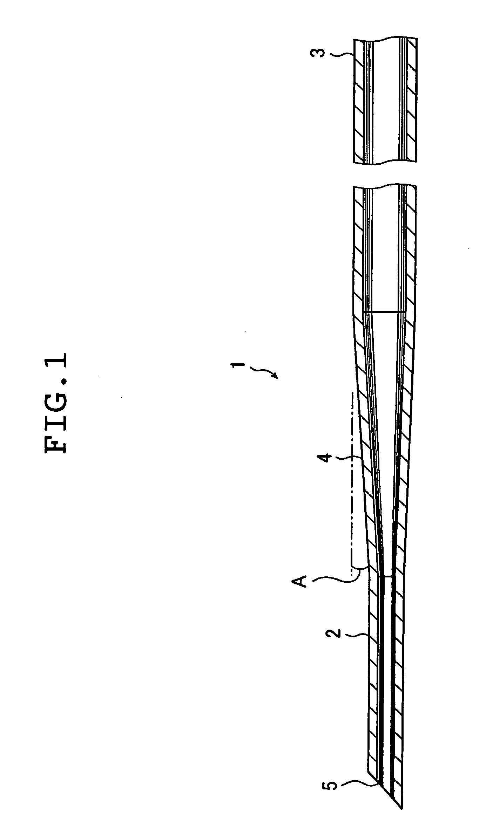

FIG. 1 is a cross-sectional view of an injection needle according to the first aspect of the present invention (hereinafter simply referred to as "injection needle according to the present invention"). The injection needle 1 comprises a hollow needle having a puncture section 2 having needle point capable of piercing a living body and a proximal end section 3 formed on a proximal end side thereof. The needle point on the puncture section 2 has a facet 5 formed by obliquely cutting the needle tube.

As indicated in a second aspect to be described later, if the injection needle 1 is used to communicate with a liquid container, then the proximal end section 3 may have a liquid introducing needle portion produced by forming a facet (i.e., the injection needle 1 is a double-pointed needle). The proximal end section 3 has inside and outside diameters greater than the puncture section 2.

The injection needle 1 also has a tapered section 4 smoothly interconnecting the puncture section 2 on the distal end side and the proximal end section 3 on the proximal end side, whose outside diameter is changed continuously from the outside diameter of the puncture section 2 to the outside diameter of the proximal end section 3.

According to the present embodiment, the outside diameter of the puncture section 2 of the injection needle 1 should preferably be in the range from 0.1 mm to 0.5 mm and more preferably be in the range from 0.1 mm to 0.3 mm. The upper limit for the outside diameter of the puncture section 2 should desirably be set so as to be smaller than the conventional outside diameter for injecting liquid medication from the standpoint of reducing puncture pain given to the patient. The lower limit therefor should desirably be set from the standpoint of maintaining predetermined mechanical strength and reducing an increase in the flow passage resistance encountered when liquid medication is injected, though the lower limit differs depending on factors such as the pierced region for injecting liquid medication, the depth by which the puncture section 2 pierces the patient, etc. Accordingly, the inside diameter of the puncture section 2 should preferably be in the range from 0.05 mm to 0.4 mm and more preferably be in the range from 0.05 mm to 0.2 mm.

The length from the puncture section 2 to the tapered section 4 of the injection needle 1 should preferably be in the range from 0.2 mm to 15 mm and more preferably be in the range from 0.5 mm to 10 mm. Generally, for hypodermic and intramuscular administration, the injection needle is inserted into the living body by a length ranging from 5 mm to 40 mm, and the length is selected depending on the location and the purpose. Therefore, the upper limit for the length from the puncture section 2 to the tapered section 4 should desirably be set from the standpoint of maintaining predetermined mechanical strength and reducing an increase in the flow passage resistance encountered when liquid medication is injected. The lower limit therefor should desirably be set from the standpoint of piercing the living body favorably as desired.

The length of the tapered section 4 should preferably be in the range from 1.5 mm to 10 mm and more preferably be in the range from 2 mm to 5 mm.

The angle of the outer profile of the tapered section 4 should preferably be in the range from 0.5 to 2 degrees with respect to a line parallel to the central axis of the injection needle 1 (i.e., the central axis of the injection needle 1). The upper limit for the angle should preferably be 1 degree and 50 minutes or less, and more preferably be 1 degree and 20 minutes or less.

Both the outer and inner profiles of the tapered section 4 should preferably be of a tapered structure.

For hypodermic and intramuscular administration, the injection needle 1 is inserted into the living body by a depth selected from between 5 mm and 40 mm depending on the location and the purpose. Therefore, if only the small-diameter puncture section 2 is to pierce the living body, then it is difficult for the injection needle 1 to keep required mechanical strength. It is also difficult for only the small-diameter puncture section 2 to provide a length required in view of injection resistance because the section with the small inside diameter would be long. Consequently, the injection needle 1 is required to have also the tapered section 4 pierce the living body.

If the puncture resistance experienced by the tapered section 4 higher than the puncture resistance experienced when the facet 5 pierces the living body, then since a larger force is needed to insert the tapered section 4 into the living body after the facet 5 has passed into the living body, it is difficult for the injection needle 1 to pierce the living body. Applying an undue force further to cause the injection needle 1 to pierce the living body tends to make the needle point move around in the living body, resulting in a high possibility to injure the living body.

Consequently, in order for the tapered section 4 not to give the patient pain and hurt and not to make the patient feel fear and anxiety, the puncture resistance experienced when the tapered section 4 pierces the living body needs to be lower than the puncture resistance experienced when the facet 5 pierces the living body. This purpose can be accomplished by forming the tapered section 4 as described above.

The outside diameter of the proximal end section 3 should preferably be in the range from 0.3 mm to 2 mm and more preferably be in the range from 0.35 mm to 1 mm. The lower limit for the outside diameter of the proximal end section 3 should desirably be set so as to be greater than the puncture section 2 as described above from the standpoint of positively reducing the flow passage resistance when liquid medication is injected into the living body. The upper limit therefor desirably be set from the standpoint of allowing the proximal end section 3 to easily pierce a resilient member if the proximal end section 3 has a liquid introducing needle section that can communicate with the interior of a liquid container sealed by a rubber plug as the resilient member. Therefore, if the injection needle 1 is used as an injection needle for drawing liquid medication from a vial which is not sealed by a resilient member, then the outside diameter of the proximal end section 3 may be greater than that of the proximal end section 3 having a liquid introducing needle to pierce the resilient member. Accordingly, the inside diameter of the proximal end section 3 should preferably be in the range from 0.25 mm to 1.9 mm.

A liquid introducing instrument according to the second embodiment of the present invention (hereinafter simply referred to as "liquid introducing instrument according to the present invention") is a liquid introducing instrument to be mounted on a liquid inlet port formed on the distal end of a liquid container that is capable of holding a liquid therein. The liquid introducing instrument comprises the injection needle according to the present invention and a base body supporting the injection needle, the puncture section and the tapered section projecting from the base body.

In the liquid introducing instrument according to the present invention, the injection needle should preferably be a double-pointed needle having a liquid introducing needle section that is capable of communicating with the interior of the liquid container.

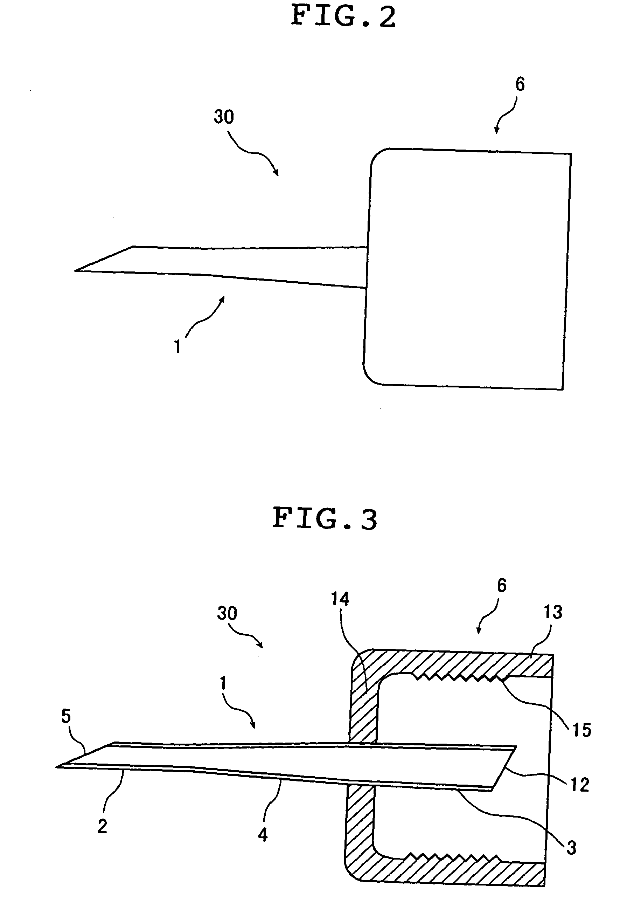

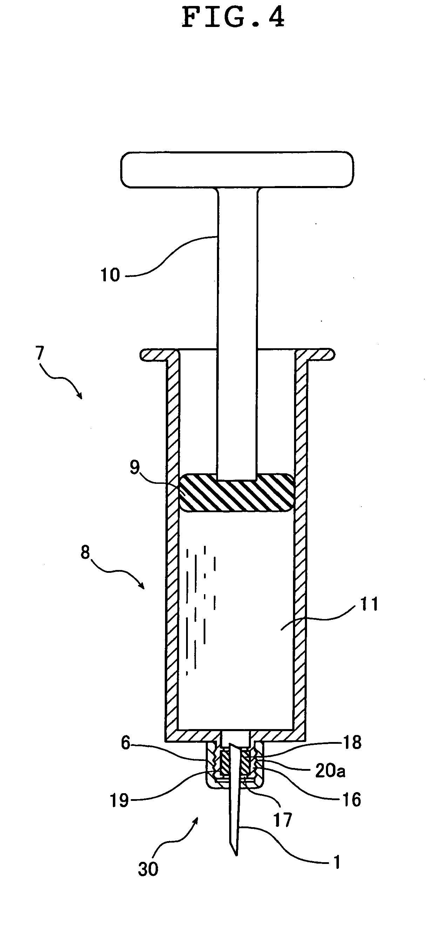

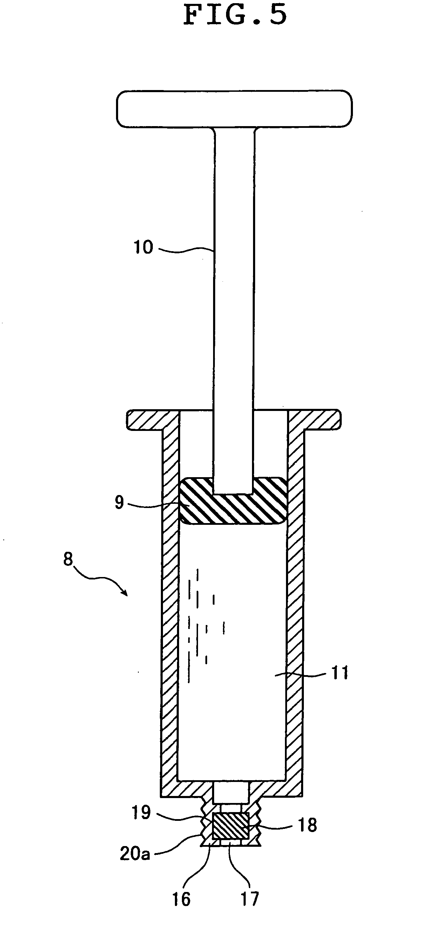

Specific examples in which the injection needle and the liquid introducing instrument according to the present invention are used will be described below with reference to FIGS. 2 through 6. FIG. 2 is a side elevational view of the liquid introducing instrument which is constructed of the injection needle according to the present invention and a base body supporting the injection needle. FIG. 3 is a cross-sectional view of the liquid introducing instrument according to the present invention shown in FIG. 2. FIG. 4 is a cross-sectional view of a liquid medication injecting device on which the liquid introducing instrument according to the present invention is mounted. FIG. 5 is a cross-sectional view of a liquid medication container shown in FIG. 4.

The liquid introducing instrument 30 comprising the injection needle 1 and a base body 6 is constructed as shown in FIGS. 2 and 3. The injection needle 1 is of the same structure as the injection needle according to the present invention, i.e., has the puncture section 2, the proximal end section 3, and the tapered section 4, with the facet 5 formed on the tip end of the puncture section 2 and a facet formed on the proximal end side of the proximal end section 3, providing a liquid introducing needle section 12. As described later on, if the tip end of a liquid medication container 8 shown in FIGS. 4 and 5 is not sealed by a resilient member, then the liquid introducing needle section 12 is not required.

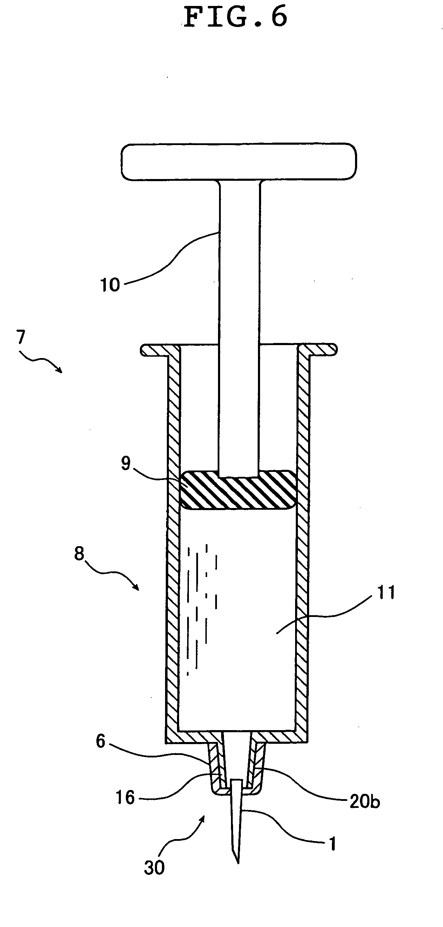

The base body 6 comprises a cylindrical wall portion 13 and a bottom wall 14 formed on an end thereof. The injection needle 1 is inserted and fixed centrally in the bottom wall 14. The cylindrical wall portion 13 has an internally threaded inner surface 15 to be threaded over an externally threaded outer surface 20a of the tip end 16 of the liquid medication container 8. If the tip end 16 of the liquid medication container 8 is of a tapered structure, then the inner surface 15 of the cylindrical wall portion 13 does not need to be internally threaded, but is tapered for fitting engagement with the tapered tip end 16 of the liquid medication container 8 (see FIG. 6).

As shown in FIGS. 2 and 3, the tapered section 4 is required to protrude wholly or in part from the bottom wall 14 in a direction away from the cylindrical wall portion 13. This is because, as described above, for hypodermic and intramuscular administration, the injection needle 1 is inserted into the living body by a depth selected from the range from 5 mm to 40 mm depending on the location and the purpose, and hence if only the small-diameter puncture section 2 is to pierce the living body, then it is difficult for the injection needle 1 to keep required mechanical strength, and it is no preferable for only the small-diameter puncture section 2 to provide a length required in view of injection resistance.

The injection needle 1 may be made of stainless steel by plastic working, for example. The injection needle 1 may also be made of metal such as titanium or the like or an alloy thereof, or a plastic material or the like. The base body 6 may be molded of a thermoplastic resin such as polypropylene, polyethylene, or the like. The injection needle 1 may be affixed to the base body 6 by an epoxy adhesive or an UV-curable adhesive, or insertion molding.

A liquid medication injecting device 7 comprises the liquid introducing instrument 30 and the liquid medication container 8, which is a container capable of holding a liquid therein. As shown in FIG. 4, the liquid medication container 8 comprises a substantially cylindrical container capable of holding liquid medication 11 therein, and has therein a gasket 9 which is slidable longitudinally in a liquid-tight fashion and a plunger 10 for pushing the gasket 9. When the plunger 10 is pushed, the liquid medication injecting device 7 injects the liquid medication 11 from the tip end of the injection needle 1 into the living body.

As shown in FIG. 5, the liquid medication container 8 has a liquid medication inlet/outlet port 17 defined in the tip end 16 thereof and sealed by a resilient member 18 such as a rubber plug, for example. The resilient member 18 is fitted in a resilient member storage cavity 19 defined as a groove in the inner surface of the tip end 16, and sealingly stores the liquid medication 11 in coaction with the gasket 9. The tip end 16, which provides the liquid inlet port defined in the tip end of the liquid medication container 8, has an externally threaded outer surface 20a for threaded engagement with the base body 6 for installing the injection needle 1.

The liquid medication container 8 should desirably be transparent wholly or partly to allow the liquid medication 11 stored therein to be visually confirmed, and should preferably be graduated for recognizing the amount of liquid medication stored therein and the amount of liquid medication injected therefrom.

Depending on the structure of the liquid medication injecting device 7 and the purpose for which it is used, the liquid medication 11 may not be sealed in the liquid medication container 8 beforehand, but may be drawn and filled in the liquid medication container 8 from a vial or the like immediately before it is injected. In such a case, the liquid medication inlet/outlet port 17 may not be sealed by a resilient member (see FIG. 6).

The outer surface of the tip end 16 may not be externally threaded, but may be in the form of a tapered surface 20b for fitting engagement with the tapered inner surface of the base body 6 (see FIG. 6).

The gasket 9 and the resilient member 18 may be made of butyl rubber, silicone rubber, an elastomer, or the like. The liquid medication container 8 and the plunger 10 may be made of a thermoplastic material such as polypropylene, polyethylene, or the like, or a material such as glass or the like.

The liquid medication 11 that is used by the liquid medication injecting device 7 comprises a solution, a gel, or a suspension including medication. The medication that can be used is not limited to particular medicinal substances, but may be, any medicinal substances insofar as they are suitable for percutaneous administration. The medication may act on local regions of the living body, or may act on the living body in its entirety. Major examples of the medication are antimicrobial, antiviral agent, vaccine, antitumor agent, immunosuppresive agent, steroidal agent, antiphlogistic agent, antirheumatic agent, drug for arthritis treatment, antihistaminic agent, antiallergenic agent, drug for diabetes treatment (insulin or the like), hormone drug, bone/calcium metabolic drug, vitamin, blood derivative, hematopoietics, antithrombotic agent, antihyperlipidemic agent, antiarrhythmic agent, vasodilator agent, prostaglandin, calcium antagonist, ACE inhibitor, β blocker, hypotensive drug, diuretic agent, xanthine derivative, β agonist, antiasthmatic agent, antitussive agent, expectorant drug, cholinolytic, stomachic digestant, antiulcer agent, cathartic, sleep promoting drug, sedative drug, antipyretic agent, cold medication, antiepileptic drug, antipsychotic agent, antidepressant agent, anxiolytic agent, analeptic agent, parasympathomimetic drug, sympathicomimetic drug, antiemetic agent, central nervous system stimulant, antiparkinson agent, muscle relaxant, spasmolytic agent, anesthetic agent, antipruritic agent, antihemicranic agent, oligonucletide, genetic drug, etc.

A process of using the injection needle and the liquid introducing instrument will be described below.

First, the liquid introducing instrument 30 is threaded and joined to the liquid medication container 8. At this time, the internally threaded inner surface 15 of the base body 6 which supports the injection needle 1 is threaded over the externally threaded outer surface 20a of the tip end 16 of the liquid medication container 8. The proximal end section 3 of the injection needle 1 that is supported on the base body 6 pierces and penetrates the resilient member 18 which seals the liquid medication inlet/outlet port 17 defined in the tip end 16 of the liquid medication container 8, providing communication between the injection needle 1 and the liquid medication container 8. The preparation for using the liquid medication injecting device 7 is now completed.

Then, the injection needle 1 is caused to pierce the skin of the patient. At this time, since the puncture section 2 is thinner than the tip ends of the conventional injection needles, the area of contact of the needle with a pain-related nerve network is reduced, lowering the simulation of the nerve network to reduce the generation of pain. The injection needle 1 is further pushed to pierce the skin to a deeper position. Since the taper angle is small, the piercing action of the injection needle 1 is smoothly carried out. All the portion of the injection needle 1 which protrudes from the base body 6 (including at least a portion of the tapered section 4) pierces the living body. By determining the length of the portion of the injection needle 1 which protrudes from the base body 6 and identifying the piercing location, a region such as an intracutaneous region, a hypodermic region, an intramuscular region, or the like is selected as a region where the liquid medication is to be injected.

The plunger 10 is pushed to inject the liquid medication 11 in the liquid medication container 8 through the injection needle 1 into a living body region such as an intracutaneous region, a hypodermic region, an intramuscular region, or any of various organs of the patient. Since the outside and inside diameters of the proximal end section 3 of the injection needle 1 are relatively large, the flow passage resistance of the injection needle 1 as a whole which is experienced at the time the liquid medication 11 is injected into the living body can be reduced. Therefore, the force needed to push the plunger 10 for forcing out the liquid medication 11 may be small, and the liquid medication 11 can well be injected into the living body.

### (Examples)

### <Injection Needle According to the Present Invention>.

An injection needle according to the present invention which is of the same shape as the injection needle 1 according to the present invention shown in FIG. 1 was fabricated. The injection needle had an overall length of 8 mm or more. Of the overall length, the length of the puncture section 2 was 2.75 mm, the length of the tapered section 4 was 3.5 mm, and the remainder was the proximal end section 3. The puncture section 2 had outside and inside diameters of 0.2 mm and 0.1 mm, respectively, and the proximal end section 3 had outside and inside diameters of 0.35 mm and 0.25 mm, respectively. At this time, the taper angle A (i.e., the angle of the outer profile of the tapered section 4) formed between the tapered surface and a line parallel to the central axis of the injection needle (i.e., the central axis of the injection needle) was 1 degree, 13 minutes, and 39 seconds.

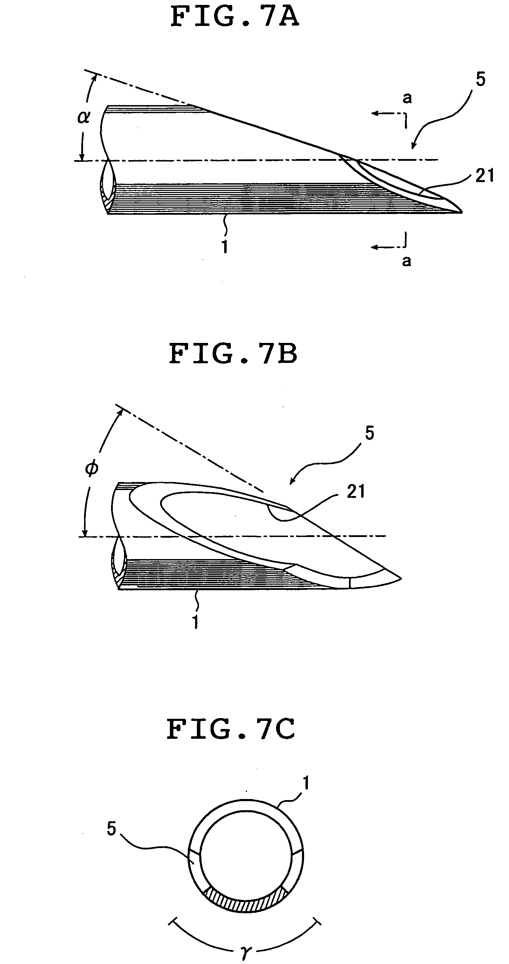

The facet 5 had a shape as shown at an enlarged scale (FIGS. 7A through 7C). A first ground angle α, a second ground angle φ, and a cross-sectional angle γ were 8.5 degrees, 18 degrees, and 129 degrees, respectively. As shown in FIG. 7A, the first ground angle α is a basic angle formed between the central axis of the injection needle 1 as indicated by the alternate long and short dash line and the slanted portion 21. As shown in FIG. 7B, the second ground angle φ is an angle formed between a cut surface providing a facet of the slanted portion 21 and the central axis. As shown in FIG. 7C, which is a cross-sectional view taken along line a-a of FIG. 7A, the cross-sectional angle γ is an angle formed between both ridges of a cross section of the needle point. (See ISO (International Organization for Standardization) 7864.)

### <Comparative Example>

As described below, an injection needle having a different taper angle A from the injection needle according to the present invention was fabricated. The injection needle had an overall length of 8 mm or more. The length of the puncture section 2 was 2.75 mm, the length of the tapered section 4 was 1 mm, and the remainder was the proximal end section 3. The puncture section 2 had outside and inside diameters of 0.2 mm and 0.1 mm, respectively, and the proximal end section 3 had outside and inside diameters of 0.35 mm and 0.25 mm, respectively. The first ground angle α, the second ground angle φ, and the cross-sectional angle γ were 8.5 degrees, 18 degrees, and 129 degrees, respectively.

At this time, the taper angle A formed between the tapered surface and a line parallel to the central axis of the injection needle (i.e., formed between the tapered surface and the central axis of the injection needle) was 4 degrees, 17 minutes, and 21 seconds.

### <Measurement of Puncture Resistance>

Puncture resistance was measured using the injection needle according to the present invention and the injection needle according to the comparative example. For measurement, the load applied when each injection needle pierces a sheet of silicone rubber at a speed of 10 mm/min. was measured and regarded as puncture resistance. The load was measured using an autograph AGS-7KNG (manufactured by Simadzu Corporation). The sheet of silicone rubber had a thickness of 0.5 mm and a hardness of A50 measured by a rubber durometer according to K6253 type A under JIS (Japan Industrial Standards).

The puncture resistance obtained by the above method represents a maximum load generated when the puncture section 2, the proximal end section 3, and the tapered section 4 pass through the sheet of silicone rubber. Though the puncture resistance is different from an actual measured value obtained when the injection needle pierces the living body, they are considered to be correlated to each other.

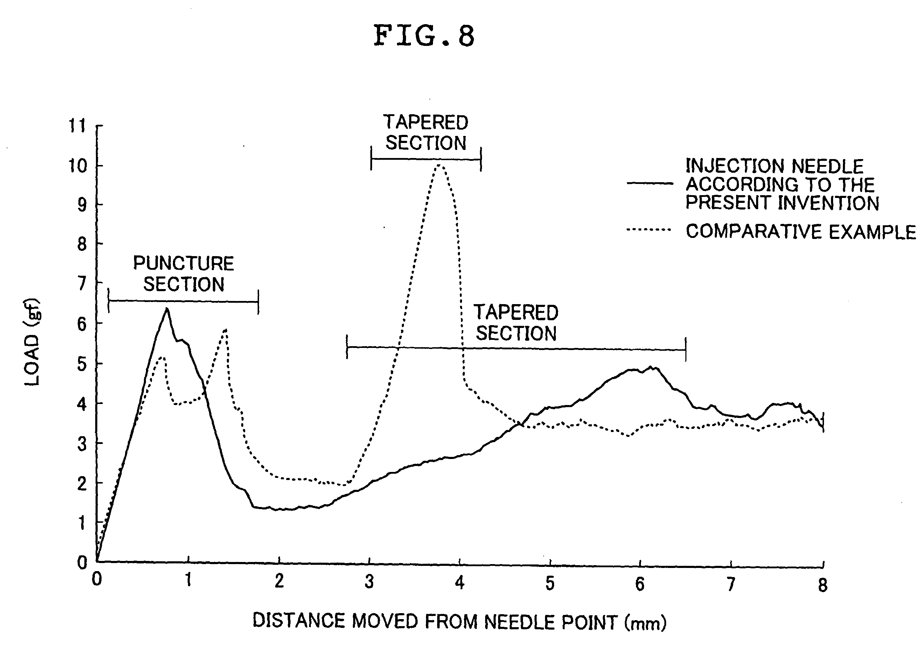

The measured results of the puncture resistance are shown in FIG. 8. The vertical axis represents the load and the horizontal axis the distance from the needle point. As shown in FIG. 8, with the injection needle according to the present invention, the puncture resistance at the tapered section 4 was of a value lower than the maximum value of the puncture resistance at the puncture section 2. Therefore, the injection needle according to the present invention can smoothly pierce the living body.

With the injection needle having a larger taper angle according to the comparative example, the puncture resistance at the tapered section was of a value much higher than the puncture resistance at the puncture section 2. Therefore, the injection needle according to the comparative example is unable to smoothly pierce the living body.

The puncture resistance of the injection needle according to the present invention increases gradually, whereas the puncture resistance of the injection needle according to the comparative example increases and decreases drastically. Therefore, the injection needle according to the present invention whose puncture resistance increases at a gradual gradient has its piercing action stabilized. From the measured results of the puncture resistance, it can be seen that the puncture resistance under the above measuring conditions at the tapered section 4 should preferably be 7 gf or less or more preferably be 6 gf or less.

### <Comparison in Use>

The injection needle according to the present invention, the injection needle according to the comparative example, and an injection needle according to a reference example 1 described below were used by healthy people (10 for each injection needle) to self-pierce their abdominal regions to a depth of 6 mm. A phenomenon in which the piercing induction needle stopped in the course of its piercing action was not confirmed with the injection needle according to the present invention and the injection needle according to the reference example 1. However, a phenomenon in which the piercing action stopped when the tapered section was about to reach the skin after the puncture section entered the intracutaneous region was confirmed with the injection needle according to the comparative example.

Therefore, a comparison between the injection needle according to the present invention and the injection needle according to the comparative example confirmed that the piercing action was smoothly performed if the puncture resistance at the tapered section was smaller than the puncture resistance at the puncture section. It was also confirmed that the piercing action was smoothly performed insofar as the puncture resistance gradually increased at the tapered section.

### <Measurement of Flow Passage Resistance>

The flow passage resistance was measured using the invention needle according to the present invention, the injection needle according to the reference example 1, and an injection needle according to a reference example 2 described below. A straight needle free of a tapered section was fabricated as the injection needle according to the reference example 1. The straight needle according to the reference example 1 had an overall length of 8 mm or more and outside and inside diameters of 0.2 mm and 0.1 mm (33 gage needle), respectively, and also had a first ground angle α of 8.5 degrees, a second ground angle φ of 18 degrees, and a cross-sectional angle γ of 129 degrees at the facet.

A straight needle free of a tapered section was fabricated as the injection needle according to the reference example 2. The straight needle according to the reference example 2 had an overall length of 8 mm or more and outside and inside diameters of 0.35 mm and 0.25 mm (31 gage needle), respectively, and also had a first ground angle α of 8.5 degrees, a second ground angle φ of 18 degrees, and a cross-sectional angle γ of 129 degrees at the facet.

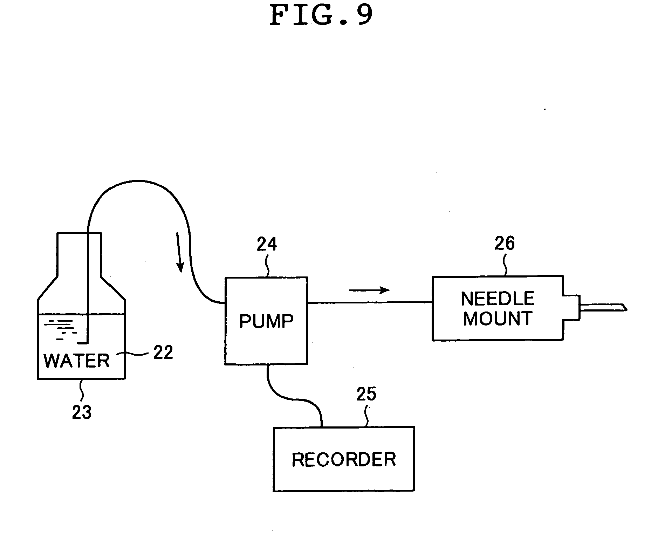

For measurement, the flow passage resistance was defined as a drive force required to keep a steady flow of water at a rate of 20 microliters/second. The measuring process was performed by a measuring system shown in FIG. 9 to record, with a recorder 25, the discharge pressure of a pump 24 in a steady state wherein water was delivered by the pump 24 at a rate of 20 microliters/second. The measuring system comprises a container 23 containing water 22, the pump 24 for delivering the water 22 under pressure which is drawn from the container 23, the recorder 25 for recording the discharge pressure of the pump 24, i.e., the drive force, and a needle mount 26 on which an injection needle to be measured is detachably mounted through threaded engagement. The water 22 is a pure water produced by way of reverse osmosis, and the pump 24 is a constant-volume pump that is generally used in high-performance liquid chromatography.

The flow passage resistance of the injection needle according to the present invention was 245 gf (2.40 N), the flow passage resistance of the injection needle according to the reference example 1 was 690 gf (6.76 N), and the flow passage resistance of the injection needle according to the reference example 2 was 272 gf (2.67 N). The flow passage resistance of the injection needle according to the present invention was one-half or less of the injection needle according to the reference example 1 though the inside diameter of the puncture section 2 was the same as that of the injection needle according to the reference example 1, and about 10 % smaller than the injection needle according to the reference example 2 though the inside diameter of the proximal end section 3 was the same as that of the injection needle according to the reference example 2.

Accordingly, it was found that the injection needle according to the present invention provides better liquid flowability than not only a straight needle free of the tapered section 4 and having an inside diameter that is the same as the inside diameter of the puncture section 2, but also a straight needle free of the tapered section 4 and having an inside diameter that is the same as the inside diameter of the proximal end section 3.

### Industrial Applicability

As described above, the injection needle and the liquid introducing instrument according to the present invention are useful because they can be used to inject liquid medication into the patient without giving the patient pain and hurt, and making the patient feel fear and anxiety. Since the flow passage resistance of the injection needle as a whole, which is encountered when injecting liquid medication into a living body, can be reduced, the injection needle and the liquid introducing instrument according to the present invention can favorably inject liquid medication into the living body, and hence are useful.

## Claims

1. An injection needle (1) comprising a puncture section (2) having a needle point (5) capable of piercing a living body, a proximal end section (3) having outside and inside diameters greater than said puncture section, and a tapered section (4) interconnecting said puncture section (2) and said proximal end section (3), said tapered section provides a puncture resistance smaller than said puncture section, said tapered section (4) has an outer profile having an angle ranging from 0 degrees 30 minutes to 1 degrees 50 minutes with respect to a line parallel to a central axis of said injection needle (1), **characterised in that** the outer diameter of the puncture section is constant and in the range from 0,1 mm to 0,5 mm, and the length of the puncture section is in a range from 0.2 mm to 15 mm.

2. The injection needle according to claim 1, wherein said tapered section (4) has outer and inner profiles each having a tapered structure.

3. A liquid introducing instrument (30) for being mounted on a liquid inlet port (16) formed on a distal end of a liquid container (8) that is capable of holding a liquid therein, comprising:
an injection needle (1) according to claim 1, and
a base body (6) supporting said injection needle (1); wherein said puncture section (2) and said tapered section (4) protrude from said base body (6).

4. The liquid introducing instrument (30) according to claim 3, wherein said injection needle (1) has a liquid introducing needle section (12) that can communicate with the interior of said liquid container (8).

## Patentansprüche

1. Injektionsnadel (1) mit einem Einstichabschnitt (2) mit einer Nadelspitze (5), die in der Lage ist, in einen lebenden Körper einzustechen, einem körpernahen Endabschnitt (3) mit einem größeren Außen- und Innendurchmesser als der Einstichabschnitt und einem sich verjüngenden Abschnitt (4), der den Einstichabschnitt (2) und den körpernahen Endabschnitt (3) miteinander verbindet, wobei der sich verjüngende Abschnitt einen geringeren Einstichwiderstand als der Einstichabschnitt vorsieht, wobei der sich verjüngende Abschnitt (4) ein Außenprofil hat, das einen Winkel aufweist, der von 0 Grad 30 Minuten bis 1 Grad 50 Minuten in Bezug auf eine Linie parallel zu einer Mittelachse der Injektionsnadel (1) reicht,
**dadurch gekennzeichnet, dass**
der Außendurchmesser des Einstichabschnitts konstant ist und sich in dem Bereich von 0,1 mm bis 0,5 mm befindet und sich die Länge des Einstichabschnitts in einem Bereich von 0,2 mm bis 15 mm befindet.

2. Injektionsnadel gemäß Anspruch 1, wobei der sich verjüngende Abschnitt (4) ein Außen- und ein Innenprofil aufweist, das jeweils einen sich verjüngende Aufbau hat.

3. Flüssigkeitseinführinstrument (30), um an einer Flüssigkeitseinlassöffnung (16) montiert zu sein, welche an einem körperfernen Ende eines Flüssigkeitsbehälters (8) ausgebildet ist, der in der Lage ist, eine Flüssigkeit in sich zu halten, mit:
einer Injektionsnadel (1) gemäß Anspruch 1 und einem Basiskörper (6), der die Injektionsnadel (1) stützt;
wobei der Einstichabschnitt (2) und der sich verjüngende Abschnitt (4) von dem Basiskörper (6) vorstehen.

4. Flüssigkeitseinführgerät (30) gemäß Anspruch 3, wobei die Injektionsnadel (1) einen Flüssigkeitseinführungsnadelabschnitt (12) hat, der mit dem Inneren des Flüssigkeitsbehälters (8) in Verbindung stehen kann.

## Revendications

1. Aiguille à injection (1), comprenant une section perforante (2) dotée d'une pointe d'aiguille (5) capable de percer le corps d'un être vivant, une section d'extrémité proximale (3) dont les diamètres externe et interne sont plus grands que ceux de ladite section perforante, et une section conique (4) qui raccorde ladite section perforante (2) et ladite section d'extrémité proximale (3), laquelle section conique (4) présente une moindre résistance de perforation que ladite section perforante et laquelle section conique (4) présente un profil extérieur qui fait un angle de 0 degré 30 minutes à 1 degré 50 minutes par rapport à une droite parallèle à l'axe central de ladite aiguille à injection (1), **caractérisée en ce que** le diamètre externe de la section perforante est constant et vaut de 0,1 mm à 0,5 mm, et la longueur de la section perforante vaut de 0,2 mm à 15 mm.

2. Aiguille à injection conforme à la revendication 1, dans laquelle ladite section conique (4) présente des profils externe et interne qui sont chacun de forme conique.

3. Instrument (30) d'introduction de liquide, conçu pour être monté sur une entrée de liquide (16) située au niveau d'une extrémité distale d'un récipient à liquide (8) capable de contenir un liquide à l'intérieur, lequel instrument comprend :
- une aiguille à injection (1), conforme à la revendication 1,
- et un corps de base (6) qui porte ladite aiguille à injection (1), et dans lequel instrument ladite section perforante (2) et ladite section conique (4) font saillie hors dudit corps de base (6).

4. Instrument (30) d'introduction de liquide, conforme à la revendi-cation 3, dans lequel ladite aiguille à injection (1) comporte une section d'introduction de liquide (12) qui peut communiquer avec l'intérieur dudit récipient à liquide (8).
